(19) Europäisches Patentamt
European Patent Office
Office européen des brevets

(11) Veröffentlichungsnummer: **0 331 030**
**A1**

(12) **EUROPÄISCHE PATENTANMELDUNG**

(21) Anmeldenummer: **89103259.1**

(22) Anmeldetag: **24.02.89**

(51) Int. Cl.4: **C07D 233/14 , C07D 233/16 , D06M 13/34 , D06M 13/46**

(30) Priorität: **04.03.88 DE 3807068**

(43) Veröffentlichungstag der Anmeldung:
**06.09.89 Patentblatt 89/36**

(84) Benannte Vertragsstaaten:
**AT BE CH DE ES FR GB GR IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Uphues, Günter**
**Robert-Koch-Strasse 45**
**D-4019 Monheim(DE)**
Erfinder: **Ploog, Uwe, Dr.**
**Haydnweg 6**
**D-5657 Haan(DE)**
Erfinder: **Becker, Wolfgang**
**Bruckneralle 224**
**D-4050 Mönchengladbach 2(DE)**
Erfinder: **Goebel, Irmhild**
**Auf dem Hennekamp 86**
**D-4000 Düsseldorf 1(DE)**

(54) **Quartäre Imidazoliniumphosphate.**

(57) Es werden quatäre Imidazoliniumphosphate beschreiben, ein Verfahren zu deren Herstellung sowie die Verwendung derselben als Antistatika.

EP 0 331 030 A1

## Quatäre Imidazoliniumphosphate

Die Erfindung betrifft quatäre Imidazoliniumphosphate, ein Verfahren zu deren Herstellung sowie die Verwendung derselben als Antistatika für Textilfasermaterialien.

Zur Vermeidung elektrostatischer Aufladungen bei der Verarbeitung sowie beim Gebrauch von Textilfasermaterialien ist eine Vielzahl antistatischer Hilfsmittel bekannt. So werden beispielsweise in "Melliand Textilberichte" 1979, 263 quartäre Ammoniummethosulfate, -chloride sowie -alkylphosphate als Antistatika beschrieben. Die Brauchbarkeit dieser Ammoniumverbindungen ist jedoch begrenzt, da die Thermostabilität von Ammoniummethosulfaten und -chloriden häufig unzureichend ist und Ammoniumalkylphosphate bei niedrigem Feuchtegehalt in der Luft keine oder nur eine sehr schwache antistatische Wirksamkeit besitzen.

In dem japanischen Schutzrecht JP 86/207 377 (Chem. Abstr. 106, 50 216 c (1987)) werden quatäre Imidazoliniumchloride und -methosulfate offenbart, die Textilmaterialien weichmachende und antistatische Eigenschaften verleihen. Die thermische Instabilität dieser Imidazoliniumsalze führt jedoch zu Vergilbungen auf Textilfasermaterialien. Insbesondere Chloride verursachen Korrosionen der Verarbeitungsaggregate. Außerdem wird zur Herstellung der Methosulfate das gesundheitsgefährdende Dimethylsulfat eingesetzt.

Die Aufgabe der vorliegenden Erfindung bestand in der Entwicklung antistatischer Mittel mit hoher antistatischer Wirksamkeit unabhängig vom Feuchtegehalt in der Luft. Ferner sollen diese antistatischen Mittel in einfacher Weise aus leicht zugänglichen Ausgangsmaterialien in Abwesenheit gesundheitsgefährdender Substanzen herstellbar und gut handhabbar sein, sich durch hohe Thermostabilitäten auszeichnen und keine korrodierende Wirkung gegenüber Metalloberflächen besitzen.

Überraschenderweise wurde gefunden, daß die an Antistatika gestellten hohen Anforderungen von Imidazoliniumphosphaten erfüllt werden, die der allgemeinen Formel I

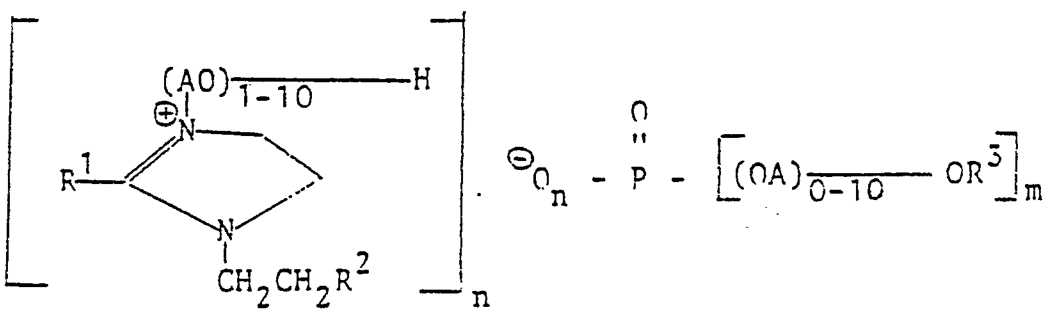

entsprechen und in der die Reste $R^1$ eine gerad- oder verzweigtkettige, gesättigte oder ungesättigte, substituierte oder unsubstituierte Alkylgruppe mit 5 bis 21 C-Atcmen, $R^2$ OH oder $-NH-\underset{O}{\underset{\|}{C}}-R^1$,

$R^3$ eine gerad-oder verzweigtkettige, gesättigte oder ungesättigte, substituierte oder unsubstituierte Alkylgruppe mit 6 bis 22 C-Atomen, A eine oder mehrere Alkylengruppen mit 2 bis 4 C-Atomen und die Indices $n = 1$ und $m = 2$ oder $n = 2$ und $m = 1$ bedeuten.

Erfindungsgegenstand sind dementsprechend quatäre Imidazoliniumphosphate der allgemeinen Formel I

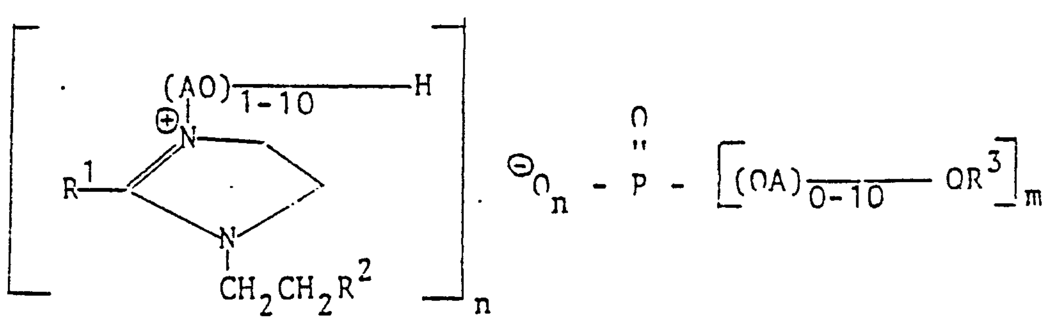

mit $R^1$ = gerad- oder verzweigtkettige, gesättigte oder ungesättigte, substituierte oder unsubstituierte

Alkylgruppe mit 5 bis 21 C-Atomen, $R^2$ = OH oder $- NH- \underset{\underset{O}{\|}}{C} -R^1$,

$R^3$ = gerad- oder verzweigtkettige, gesättigte oder ungesättigte, substituierte oder unsubstituierte Alkylgruppe mit 6 bis 22 C-Atomen, A = Alkylengruppen mit 2 bis 4 C-Atomen und n, m = 1 oder 2 mit der Maßgabe, daß n ungleich m ist.

Bevorzugt werden solche Imidazoliniumphosphate der allgemeinen Formel I, in der die Reste $R^1$ eine gerad- oder verzweigtkettige, substituierte oder unsubstituierte, gesättigte Alkylgruppe mit 7 bis 17 C-Atomen oder eine gerad- oder verzweigtkettige, substituierte oder unsubstituierte, ungesättigte Alkylgruppe mit 17 bis 21 C-Atomen, $R^2$ OH, $R^3$ eine gerad- oder verzweigtkettige, substituierte oder unsubstituierte, gesättigte Alkylgruppe mit 8 bis 18 C-Atomen oder eine gerad- oder verzweigtkettige, substituierte oder unsubstituierte, ungesättigte Alkylgruppe mit 18 bis 22 C-Atomen und A Ethylen- und/oder Isopropylengruppen darstellen.

Ein weiterer Erfindungsgegenstand ist ein Verfahren zur Herstellung von quatären Imidazoliniumphosphaten der allgemeinen Formel I

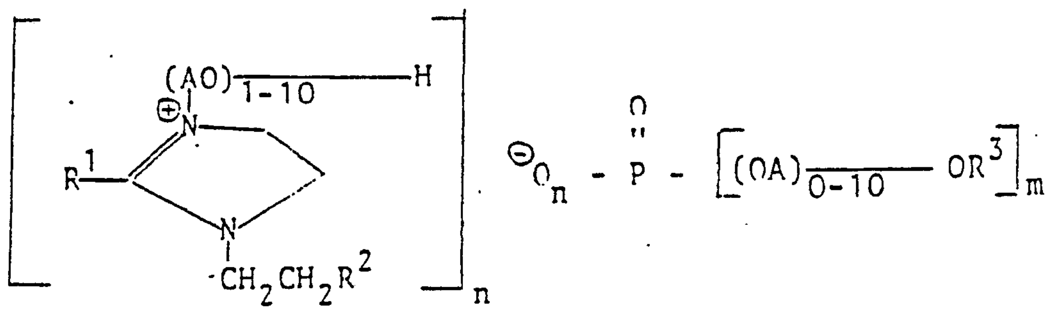

in der die Reste $R^1$ eine gerad- oder verzweigtkettige, gesättigte oder ungesättigte, substituierte oder unsubstituierte Alkylgruppe mit 5 bis 21 C-Atomen, $R^2$ OH oder $-NH- \underset{\underset{O}{\|}}{C} -R^1$,

$R^3$ eine gerad- oder verzweigtkettige, gesättigte oder ungesättige, substituierte oder unsubstituierte Alkylgruppe mit 6 bis 22 C-Atomen und A Alkylengruppen mit 2 bis 4 C-Atomen darstellen, und die Indices n und m die Zahl 1 oder 2 bedeuten mit der Maßgabe, daß n ungleich m ist, welches dadurch gekennzeichnet ist, daß Imidazoline der allgemeinen Formel II

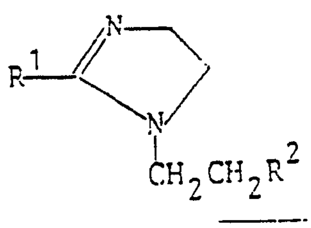

in der die Reste $R^1$ und $R^2$ die für die Formel I angegebene Bedeutung haben, und Mono- und/oder Dialkylphosphorsäureester der allgemeinen Formeln III a und III b

IIIa

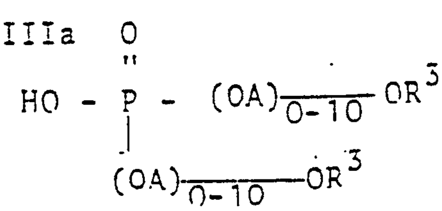

IIIb

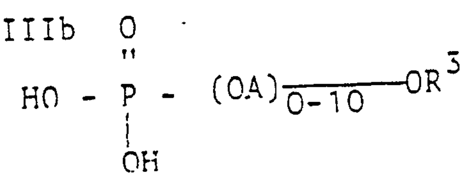

mit $R^3$ = gerad- oder verzweigtkettige, gesättigte oder ungesättigte, substituierte oder unsubstituierte Alkylgruppen mit 6 bis 22 C-Atomen und A = Alkylengruppen mit 2 - 4 C-Atomen in Gegenwart von Wasser mit 3 bis 15 Mol $C_{2-4}$-Alkylenoxiden pro Mol Imidazolin bei Drucken von 1 bis 5 bar und Temperaturen von 80 bis 100 °C umgesetzt werden.

Für das erfindungsgemäße Verfahren werden bevorzugt solche Imidazoline der allgemeinen Formel II und solche Mono- und/oder Dialkylphosphorsäureester der allgemeinen Formeln III a und IIIb eingesetzt, in welchen die Reste $R^1$ eine gerad- oder verzweigtkettige, substituierte oder unsubstituierte, gesättigte Alkylgruppen mit 7 bis 17 C-Atomen oder eine gerad- oder verzweigtkettige, substituierte oder unsubstituierte, ungesättigte Alkylgruppen mit 17 bis 21 C-Atomen, $R^2$ OH, $R^3$ eine gerad-oder verzweigtkettige, substituierte oder unsubstituierte, gesättigte Alkylgruppe mit 8 bis 18 C-Atomen oder eine gerad-oder verzweigtkettige, substituierte oder unsubstituierte, ungesättigte Alkylgruppe mit 18 bis 22 C-Atomen und A Ethylen-und/oder Isopropylengruppen bedeuten.

Zur Herstellung der Imidazoliniumphosphate der allgemeinen Formel I werden Mischungen aus Imidazolinen, Mono- und/oder Dialkylphosphorsäureestern, Wasser und gegebenenfalls polaren organischen Lösungsmitteln bereitet, wobei pro Mol Imidazolin vorzugsweise 1 bis 1,1 Säureäquivalente eingesetzt werden. Als polare organische Lösungsmittel werden insbesondere aliphatische Alkohole mit 1 bis 6 C-Atomen beispielsweise Methanol, Ethanol, n-Propanol, i-Propanol, n-Butanol, n-Hexanol, Ethylenglycol, Propylenglycol und/oder Hexylenglycol eingesetzt, wobei das Volumenverhältnis Wasser zu organischem Lösungsmittel zwischen 10 : 1 und 1 : 4 liegt. Die Menge an Wasser, gegebenenfalls in Kombination mit polaren organischen Lösungsmitteln wird vorzugsweise so bemessen, daß der Gehält quatärer Imidazoliniumphosphate in den Produkten zwischen 30 und 80 Gewichtsprozent liegt.

In diese Gemische, enthaltend Imidazoline, Mono- und/oder Dialkylphosphorsäurester, Wasser und gegebenenfalls polare organische Lösungsmittel, werden anschließend in an sich bekannter Weise Alkylenoxide mit 2 bis 4 C-Atomen bei Drucken zwischen 1 und 5 bar und Temperaturen zwischen 80 und 100 °C eingeleitet, mit der Maßgabe, daß das Molverhältnis Imidazolin: Alkylenoxid zwischen 1 : 3 und 1 : 15 liegt. Die Umsetzungen werden vorzugsweise mit Ethylenoxid und/oder Propylenoxid durchgeführt.

Die als Edukte zur Herstellung von Imidazoliniumphosphaten der allgemeinen Formel I benötigten Imidazoline können durch Kondensation von Carbonsäuren, Carbonsäureestern oder Carbonsäurechloriden mit Diaminverbindungen hergestellt werden (G. Gawalek: "Tenside" S. 305 - 306, Akademie-Verlag Berlin 1975). Vorzugsweise erfolgt die Herstellung der Imidazoline der allgemeinen Formel II, wie in der deutschen Offenlegungsschrift DE 36 18 944 beschrieben, durch Kondensation von Carbonsäuren der allgemeinen Formel IV

$R^1$ - COOH,

in der $R^1$ eine gerad- oder verzweigtkettige, gesättigte oder ungesättigte, substituierte oder unsubstituierte Alkylgruppe mit 5 bis 21 C-Atomen bedeutet, mit Diaminen der allgemeinen Formel V

$H_2N\text{-}CH_2CH_2\text{-}NH\text{-}CH_2CH_2R^2$,

in der $R^2$ OH oder $-NH-\underset{\underset{O}{\|}}{C}-R^1$

bedeutet.

Zur Herstellung der Imidazoline können die Carbonsäuren sowohl einzeln als auch im Gemisch eingesetzt werden. Von besonderer Bedeutung ist der Einsatz von Fettsäuregemischen, wie sie bei der Spaltung natürlich vorkommender Fette und/oder Öle anfallen.

Einzelne Fraktionen solcher Fettsäuregemische eignen sich ebenfalls zur Herstellung der Imidazoline. Als Carbonsäuren werden vorzugsweise solche der allgemeinen Formel IV eingesetzt, in der der Rest $R^1$ für eine gerad- oder verzweigtkettige, substituierte oder unsubstituierte, gesättige Alkylgruppe mit 7 bis 17 C-Atomen oder für eine gerad- oder verzweigtkettige, substituierte oder unsubstituierte, ungesätttigte Alkylgruppe mit 17 bis 21 C-Atomen steht, beispielsweise Caprylsäure, Caprinsäure, Laurinsäure, Myristinsäure, Palmitinsäure, Stearinsäure, Ölsäure, Linolsäure, Linolensäure, Gadoleinsäure und/oder Rizinolsäure.

Die Carbonsäuren der allgemeinen Formel IV werden mit den Diaminverbindungen der allgemeinen Formel V bei Temperaturen zwischen 150 und 240 °C, vorzugsweise zwischen 150 und 200 °C kondensiert, wobei das Molverhältnis Carbonsäure : Diamin vorzugsweise zwischen 1 : 1 und 1 : 2 liegt. Das während der Kondensation gebildete Wasser wird kontinuierlich mittels Destillation, gewünschtenfalls in Gegenwart von Lösungsmitteln, die mit Wasser Azeotrope bilden, beispielsweise Toluol der Xylol, aus dem Reaktionsgemisch entfernt.

Die zur Herstellung von Imidazoliniumphosphaten der allgemeinen Formel I benötigte zweite Ausgangskomponente, die Mono-und/oder Dialkylphosphorsäureester, ist ebenfalls nach literaturbekannten Verfahren zugänglich. Die Herstellung der Phosphorsäurepartialester geht aus von gerad- oder verzweigtkettigen, gesättigten oder ungesättigten, substituierten oder unsubstituierten Alkoholen mit 6 bis 22 C-Atomen und/oder deren Alkoxylaten. Vorzugsweise werden gerad- oder verzweigtkettige, substituierte oder unsubstituierte, gesättigte, gewünschtenfalls alkoxylierte Alkohole mit 8 bis 18 C-Atomen und/oder gerad- oder verzweigtkettige, substituierte oder unsubstituierte, ungesättigte, gewünschtenfalls alkoxylierte Alkohole mit 18 bis 22 C-Atomen eingesetzt, beispielsweise 2-Ethylhexanol, n-Octanol, Isononyl-, Decyl-, Dodecyl-,

Isotridecyl-, Tetradecyl-, Hexadecyl-, Octadecyl-, Octadecenyl-, Eicosenyl-, Docosenylalkohol sowie die entsprechenden mit 1 - 10 Mol Ethylen- und/oder Propylenoxid umgesetzten Alkohole. Die Phosphatierung der Alkohole erfolgt in an sich bekannter Weise, in dem beispielsweise 3 Mol Alkohole mit 1 Mol $P_2O_5$ bei 50 - 80° C umgesetzt werden. Zur Hydrolyse von Polyphosphorsäureverbindungen wird anschließend mehrere Stunden bei 90 - 100° C mit Wasser behandelt.

Die nach dem erfindungsgemäßen Verfahren in hohen Ausbeuten zugänglichen Imidazoliniumphosphate der allgemeinen Formel I fallen als gelbe bis hellbraune, niedrigviskose Flüssigkeiten an, die ohne Gelbildung mit Wasser mischbar sind. Sie besitzen gegenüber Textilfasermaterialien eine hohe antistatische Wirkung, die vom Feuchtegehalt in der Luft weitgehend unabhängig ist. Des weiteren zeichnen sich die erfindungsgemäßen Verbindungen durch hohe Thermostabilitäten aus und zeigen keine korrodierende Wirkung gegenüber Metalloberflächen.

Die Verwendung von Imidazoliniumphosphaten der allgemeinen Formel I als Antistatika für Textilfasermaterialien ist daher ebenfalls Erfindungsgegenstand.

Unter dem Begriff "Textilfasermaterialien" werden natürliche Fasern, beispielsweise Baumwolle, Wolle und/oder Zellwolle, synthetische Fasern, beispielsweise Polyacrylnitril, Polyester, Polyamid, Triacetat, Polyethylen und/oder Polypropylen sowie Mischungen natürlicher und synthetischer Fasern verstanden. Die Textilfasermaterialien können als Flocke, Kammzug, Strickgarn, Webgarn, Nähgarn, Wirkware, Gewebe oder Nonwovens vorliegen. Das Material kann rohweiß, gebleicht oder gefärbt sein. Bei den Garnen kann es sich sowohl um Filament- als auch um Fasergarne handeln.

Die erfindungsgemäßen quatären Imidazoliniumphosphate werden mit Hilfe üblicher Apparaturen auf die Textilfasermaterialien appliziert. Die Applikation kann nach einem Ausziehverfahren, beispielsweise auf Färbeapparaten, aus wäßriger Flotte mit einem Flottenverhältnis zwischen 1 : 5 und 1 : 40 und einem pH-Wert in der Flotte zwischen 3,0 und 7,0 bei Temperaturen zwischen 25 und 80 °C und einer auf das Warengewicht bezogenen Konzentration an erfindungsgemäßen Imidazoliniumphosphaten von 1 bis 5 Gew.% erfolgen. Die quatären Imidazoliniumphosphate können jedoch auch nach einem Zwangsapplikationsverfahren, beispielsweise mittels Foulardieren, Galettenapplikation, über Dosierpumpensysteme oder durch Aufsprühen auf die Textilfasermaterialien aufgebracht werden. Im Foulardverfahren wird in Abhängigkeit vom Gewünschten Abquetscheffekt sowie von der gewünschten Auflage mit einer Konzentration von 1 - 50 g/l Flotte - bezogen auf 100 % Wirkstoffgehalt - gearbeitet.

## Beispiele

### Herstellung von Imidazoliniumphosphaten

126 g (0,47 Mol) 1-Hydroxyethyl-2-undecylimidazolin wurden zusammen mit 80,0 g Wasser und 88,4 g (0,47 Säureäquivalente) eines Gemisches aus Mono- und Di-2-ethylhexylphosphorsäureester (Gewichtsverhältnis 4 : 3) in einem Autoklaven vorgelegt und innerhalb von 4 Stunden mit 103,4 g (2,35 Mol) Ethylenoxid bei 80 °C / 3 bar zur Reaktion gebracht.

Es wurden 395 g einer gelben, niedrigviskosen, ohne Gelbildung mit Wasser verdünnbaren Flüssigkeit A erhalten.

### Prüfergebnisse

1. Applikation im Ausziehverfahren

Gewebeproben aus Polyester- und Polyacrylnitrilfasern wurden bei einem Flottenverhältnis von 1 : 20 20 Minuten in eine 40 - 45° C warme wäßrige Lösung getaucht, die bezogen auf das Warengewicht 3 Gew.-% einer Ammoniumverbindung enthielt. Danach wurde geschleudert und 3 Minuten bei 120 °C getrocknet.

Die bei 22 °C und 62 - 65 % relative Luftfeuchte (Klima 1) sowie bei 23 °C und 25 % relative Luftfeuchte (Klima II) klimatisierten Gewebeproben wurden wie folgt geprüft:

a) auf einem Statikvoltmeter (Messung der Reibungsaufladung); es wird die Feldzerfallhalbwertszeit (FHZ) ermittelt. FHZ ist die Zeit in Sekunden, in der die sofort nach dem Reiben an Stahl gemessene Ladung auf die Hälfte abgesunken ist.

b) mit einer Siemenselektrode auf einem Teraohmmeter (elektrischer Oberflächenwiderstand in Ohm).

| Bewertungsschema | | | |
|---|---|---|---|
| | | antistatischer Effekt | |
| | | FHZ (sec.) | Oberflächenwiderstand (Ohm) |
| + + + | sehr gut | 0 - 0,3 | $1 \times 10^6$ - $1 \times 10^8$ |
| + + | gut | 0,3 - 1,0 | $1 \times 10^8$ - $1 \times 10^9$ |
| + | befriedigend | 1,0 - 2,0 | $1 \times 10^9$ - $1 \times 10^{10}$ |
| 0 | ausreichend | 2,0 - 3,0 | $1 \times 10^{10}$- $5 \times 10^{10}$ |
| - | ungenügend | > 3,0 | > $5 \times 10^{10}$ |

Tabelle

| Ergebnisse | | | | | |
|---|---|---|---|---|---|
| Gewebe | Substanz | Klima I | | Klima II | |
| | | FHZ | Ohm | FHZ | Ohm |
| Polyester | unbehandelt | - | - | - | - |
| Polyester | A | + + + | + + | + | o |
| Polyacrylnitril | unbehandelt | - | - | - | - |
| Polyacrylnitril | A | + + + | + + | + + | o |

2. Zwangsapplikation nach dem Foulard-Verfahren

Gewebeproben aus Polyester-, Polyamid- und Polyacrylnitrilfasern wurden foulardmäßig wie folgt behandelt:

| | |
|---|---|
| Flottentemperatur: | 25 °C |
| Flottenaufnahme: | 100 Gew.-% bei Polyester<br>65 Gew.-% bei Polyamid<br>120 Gew.-% bei Polyacrylnitril |

Produktauflagen: 0,25 Gew.-%, 0,5 Gew.-%, 1,0 Gew.% und 2,0 Gew.-%

Danach wurde 3 Minuten bei 120 °C getrocknet. Zusätzlich wurden die getrockneten Gewebe geteilt und im Fall des Polyesters bei 180 °C und im Fall des Polyamids bei 190 °C jeweils 30 Sekunden fixiert. Prüfung und Bewertung erfolgten wie unter 1.) beschrieben.

Tabelle

| Ergebnisse | | | | | | | | | |
|---|---|---|---|---|---|---|---|---|---|
| Klima I | | | | | | | | | |
| Gewebe | Substanz | Produktauflage | | | | | | | |
| | | 0,25% | | 0,5% | | 1,0% | | 2,0% | |
| | | FHZ | Ohm | FHZ | Ohm | FHZ | Ohm | FHZ | Ohm |
| Polyester | unbehandelt | - | - | - | - | - | - | - | - |
| Polyester | A | + + + | + + + | + + + | + + + | + + + | + + | + + + | + + |
| Polyester | A,fixiert | + + + | + + + | + + + | + + + | + + + | + + | + + + | + + |
| Polyamid | unbehandelt | - | - | - | - | - | - | - | - |
| Polyamid | A | o | o | + + | o | + + | + | + + + | + |
| Polyamid | A,fixiert | - | o | - | o | + | + | + + + | + |
| Polyacrylnitril | unbehandelt | - | - | - | - | - | - | - | - |
| Polyacrylnitril | A | + + + | + + | + + + | + + | + + + | + + | + + + | + + |
| Klima II | | | | | | | | | |
| Polyester | unbehandelt | - | - | - | - | - | - | - | - |
| Polyester | A | + + | + | + + | + | + + | + | + + + | + |
| Polyester | A,fixiert | + + | o | + + | o | + + | + | + + + | + |
| Polyamid | unbehandelt | - | - | - | - | - | - | - | - |
| Polyamid | A | - | - | - | - | o | o | + | o |
| Polyamid | A, fixiert | - | - | - | - | o | o | + | o |
| Polyacrylnitril | unbehandelt | - | - | - | - | - | - | - | - |
| Polyacrylnitril | A | + | + | + + | + | + + | + | + + | + |

**Ansprüche**

1. Quatäre Imidazoliniumphosphate der allgemeinen Formel I

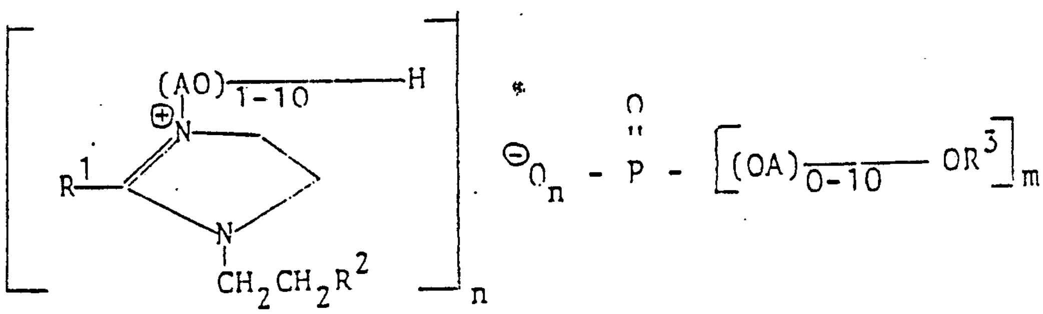

in der die Reste $R^1$ gerad- oder verzweigtkettige, gesättigte oder ungesättigte, substituierte oder unsubstituierte Alkylgruppen mit 5 bis 21 C-Atomen, $R^2$ OH oder $-NH-\underset{\overset{\|}{O}}{C}-R^1$,

$R^3$ gerad- oder verzweigtkettige, gesättigte oder ungesättigte, substituierte oder unsubstituierte Alkylgruppen mit 6 bis 22 C-Atomen und A Alkylengruppen mit 2 bis 4 C-Atomen darstellen, die Indices n und m 1 oder 2 bedeuten, mit der Maßgabe, daß n ungleich m ist.

2. Quatäre Imidazoliniumphosphate nach Anspruch 1, dadurch gekennzeichnet, daß die Reste $R^1$ eine gerad-oder verzweigtkettige, substituierte oder unsubstituierte, gesättigte Alkylgruppe mit 7 bis 17 C-Atomen oder eine gerad- oder verzweigtkettige, substituierte oder unsubstituierte, ungesättigte Alkylgruppe mit 17 bis 21 C-Atomen, $R^2$ OH, $R^3$ eine gerad- oder verzweigtkettige, substituierte oder unsubstiuierte,

gesättigte Alkylgruppe mit 8 bis 18 C-Atomen oder eine gerad- oder verzweigtkettige, substituierte oder unsubstituierte, ungesättigte Alkylgruppe mit 18 bis 22 C-Atomen und A Ethylen- und/oder Isopropylengruppen bedeuten.

3. Verfahren zur Herstellung von quatären Imidazoliniumphosphaten der allgemeinen Formel I

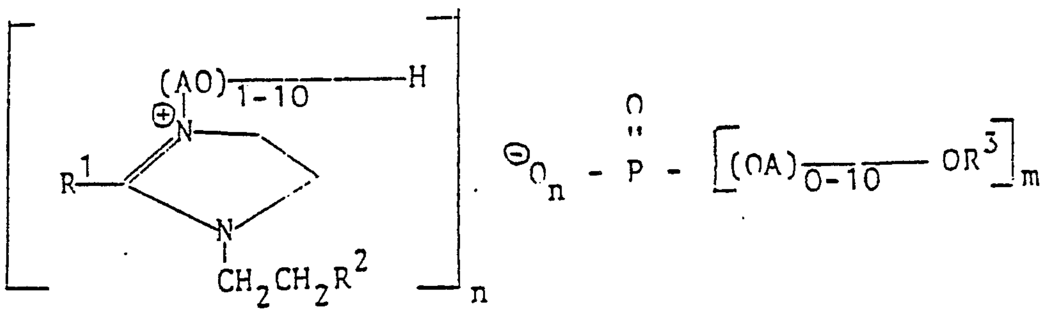

in der die Reste $R^1$ gerad- oder verzweigtkettige, gesättigte oder ungesättigte, substituierte oder unsubstituierte Alkylgruppen mit 5 bis 21 C-Atomen, $R^2$ OH oder $-NH-\underset{\ddot{O}}{C}-R^1$,

$R^3$ gerad- oder verzweigtkettige, gesättigte oder ungesättigte, substituierte oder unsubstituierte Alkylgruppen mit 6 bis 22 C-Atomen und A Alkylengruppen mit 2 bis 4 C-Atomen darstellen, die Indices n und m 1 oder 2 bedeuten mit der Maßgabe, das n ungleich m ist, dadurch gekennzeichnet, daß Imidazoline der allgemeinen Formel II

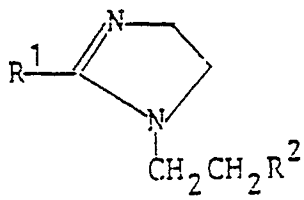

in der die Reste $R^1$ und $R^2$ die für die allgemeine Formel I angegebene Bedeutung haben und Mono- und/oder Dialkylphosphorsäureester der allgemeinen Formel IIIa und IIIb

IIIa

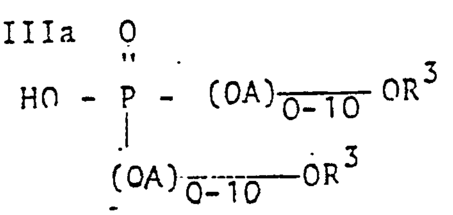

IIIb

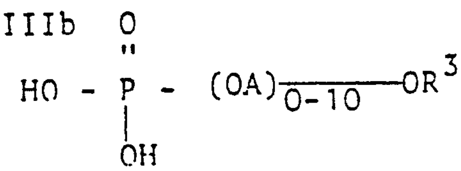

in denen $R^3$ = gerad- oder verzweigtkettige, gesättigte oder ungesättigte, substituierte oder unsubstituierte Alkylgruppen mit 6 bis 22 C-Atomen und A Alkylengruppen mit 2 - 4 C-Atomen bedeutet, in Gegenwart von Wasser mit 3 bis 15 Mol $C_{2-4}$-Alkylenoxiden pro Mol Imidazolin bei Drucken von 1 bis 5 bar und Temperaturen zwischen 80 und 100 °C umgesetzt werden.

4. Verfahren nach Anspruch 3, dadurch gekennzeichnet, daß in den allgemeinen Formeln II, IIIa und IIIb die Reste $R^1$ eine gerad- oder verzweigtkettige, substituierte oder unsubstituierte, gesättigte Alkylgruppe mit 7 bis 17 C-Atomen oder eine gerad- oder verzweigtkettige, substituierte oder unsubstituierte, ungesättigte Alkylgruppe mit 17 bis 21 C-Atomen, $R^2$ OH, $R^3$ eine gerad- oder verzweigtkettige, substituierte oder unsubstituierte, gesättigte Alkylgruppe mit 8 bis 18 C-Atomen oder eine gerad- oder verzweigtkettige, substituierte oder unsubstituierte, ungesättigte Alkylgruppe mit 18 bis 22 C-Atomen und A Ethylen-und/oder Isopropylengruppen darstellen.

5. Verfahren nach Anspruch 3 und/oder Anspruch 4, dadurch gekennzeichnet, daß pro Mol Imidazolin 1 bis 1,1 Säureäquivalente an Phosphorsäureester eingesetzt werden.

6. Verfahren nach einem oder mehreren der Ansprüche 3 bis 5, dadurch gekennzeichnet, daß die Umsetzungen mit Ethylenoxid und/oder Propylenoxid durchgeführt werden.

7. Verwendung von quatären Imidazoliniumphosphaten der allgemeinen Formel I

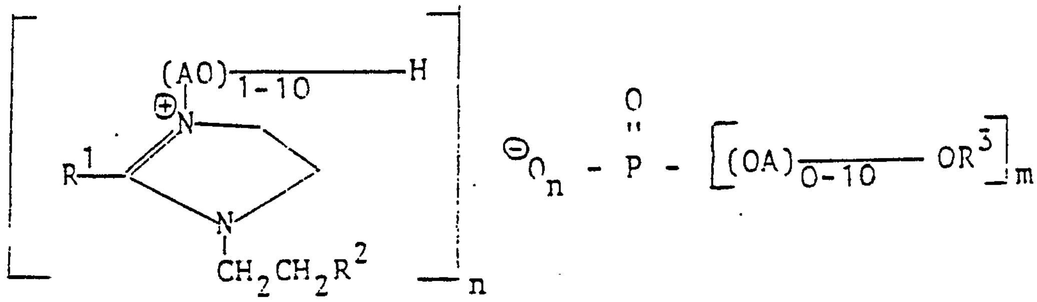

in der die Reste $R^1$ gerad- oder verzweigtkettige, gesättigte oder ungesättigte, substituierte oder unsubstituierte Alkylgruppen mit 5 bis 21 C-Atomen, $R^2$ OH oder $-NH-\underset{\overset{\|}{O}}{C}-R^1$,

$R^3$ gerad- oder verzweigtkettige, gesättigte oder ungesättigte, substituierte oder unsubstituierte Alkylgruppen mit 6 bis 22 C-Atomen und A Alkylengruppen mit 2 bis 4 C-Atomen darstellen und die Indices n und m 1 oder 2 bedeuten mit der Maßgabe, das n ungleich m ist, als Antistatika für Textilfasermaterialien.

Europäisches Patentamt

# EUROPÄISCHER RECHERCHENBERICHT

Nummer der Anmeldung

EP 89 10 3259

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 248 365 (HENKEL KGAA)<br>* Ansprüche 1,23 *; & DE - A - 30 18944 (Kat. D)<br>--- | 1,7 | C 07 D 233/14<br>C 07 D 233/16<br>D 06 M 13/34<br>D 06 M 13/46 |
| D,Y | MELLIAND TEXTILBERICHTE Band 60, 1979, Seiten 263-267, Heidelberg, D; R. KLEBER:"Neuentwicklungen der Faserpräparations- und Avivagechemie" * Seite 263, Abschnitt 1.1 *<br>--- | 1,7 | |
| Y | DE-A-2 833 172 (CIBA-GEIGY AG)<br>* Ansprüche 1-3,7,21 *<br>--- | 1,7 | |
| A | PATENT ABSTRACTS OF JAPAN Band 11, Nr. 43 (C-402)(2490), 7. Februar 1987; & JP - A- 61 207 377 (KAD CORP.) 13.09.1986 (Kat. D,A)<br>--- | 1,7 | |
| A | EP-A-0 075 168 (BAYER AG)<br>* Zusammenfassung *<br>--- | 1,7 | **RECHERCHIERTE SACHGEBIETE (Int. Cl.4)** |
| A | GB-A-1 427 094 (HOECHST AG)<br>* gesamtes Dokument *<br>----- | 1,7 | C 07 D 233/00<br>D 06 M 13/00 |

Der vorliegende Recherchenbericht wurde für alle Patentansprüche erstellt

| Recherchenort | Abschlußdatum der Recherche | Prüfer |
|---|---|---|
| BERLIN | 30-05-1989 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument